# EUROPEAN PATENT APPLICATION

(11) **EP 2 444 808 A1**
(43) Date of publication of application: **25.04.2012**
(21) Application number: 10188398.1
(22) Date of filing: 21.10.2010
(51) Int. Cl.: G01N 33/543

(54) **Method for fusion of lipid bilayers**

(71) Applicant: Höök, Fredrik, 441 43 Alingsås (SE); Simonsson, Lisa, 411 31 Göteborg (SE); Gunnarsson, Anders, 416 58 Göteborg (SE); Roca, Mateus Pla, 8046 Zürich (CH); Reimhult, Erik Olof, 1220 Wien (AT); Jönsson, Peter, 412 81 Göteborg (SE)
(72) Inventor: Höök, Fredrik, 441 43 Alingsås (SE); Simonsson, Lisa, 411 31 Göteborg (SE); Gunnarsson, Anders, 416 58 Göteborg (SE); Roca, Mateus Pla, 8046 Zürich (CH); Reimhult, Erik Olof, 1220 Wien (AT); Jönsson, Peter, 412 81 Göteborg (SE)
(74) Representative: Jönsson, Anneli

(57) **Abstract**

A method for providing a lipid bilayer is disclosed, comprising the steps of providing a first and a second lipid bilayer on a surface of a supportive substrate, and providing a force such that the first lipid bilayer is driven along the surface of the substrate towards said second lipid bilayer and said first lipid bilayer is fused with said second lipid bilayer. The method facilitates fusion of lipid bilayers and generation of surface supported lipid bilayers.

## Description

### FIELD OF THE INVENTION

The present invention relates to a method for providing a surface supported lipid bilayer.

### BACKGROUND OF THE INVENTION

Surface supported lipid bilayers have for several decades served as important model systems of the natural cell membrane in fundamental research, medical diagnostics, biotechnology, cell membrane proteomics and pharmaceutical development. This stems from the detailed information on the physical and chemical properties of cell membranes that can be obtained using sensors that are compatible with surface supported lipid bilayers, such as, but not limited to techniques sensitive to interfacial refractive index contrasts, including surface plasmon resonance and ellipsometry, gravimetric techniques, including quartz crystal microbalance devices and surface acoustic wave devices, electrochemical, patch clamp or electrical impedance spectroscopy devices, scanning probe techniques, mass spectrometry techniques, fluorescence readout principles and techniques based on optical interference including dual polarization interferometry.

One example that demonstrates the importance of surface supported cell membranes is the significant efforts that have been devoted to separate and isolate membrane-associated molecules [US2004018601 (A1); WO2009132124 (A2); US2003102263 (A1)]. In contrast to water-soluble biomolecules, for which chromatographic methods have long been established, the separation and identification of membrane-associated molecules has been identified as a significantly more demanding task. The main problem associated with membrane protein separation and isolation with existing methods is that the molecules must be extracted from the lipid bilayer using detergents before they can be purified by conventional chromatography, a procedure that can damage or alter the structure and function of the molecules.

Methods of separating membrane-associated molecules in a lipid environment have so far primarily utilized electric fields to drive charged molecules in continuous surface supported lipid bilayers. This has allowed the separation of different types of charged lipids as well as membrane-associated proteins [Daniel, S. et al JACS, 2007, (129) p8072; Groves, J.T. et al, Biophys. J., 1996, (71) p2716]. Alternative approaches that do not rely on the charge of the molecules is the self-spreading approach or the use of surface acoustic waves [Neumann, J. et al, Nano Letters, 2010, (10) p2903].

Another example is the use of surface supported lipid bilayers as cell membrane mimics for studies of molecular recognition reactions.

A further another example is the use of surface supported cell membranes to steer and control the behaviour of cells attached to the surface supported bilayer via molecular recognition reactions[WO2007084962 (A2)].

One of the most common strategies to form surface supported lipid bilayers is via liposome adsorption, which on certain surfaces leads to spontaneous surface supported lipid bilayer formation. Due to a delicate balance between liposome-surface and liposome-liposome interactions, the lipid compositions of liposomes that promote spontaneous surface supported lipid bilayer formation are limited, and the process is generally restricted to only a handful surface materials, including SiO₂, Si₃N₄, mica and chemically modified gold. Surface supported liposomes or dissolved lipids can also, under certain conditions, be converted to a surface supported lipid bilayer on gold and TiO₂ upon addition of certain peptides [WO2006110350]. An alternative, but more time consuming, way of forming surface supported lipid bilayers is via so called Langmuir-Blodgett deposition, in which case lipids are transferred to a substrate from an air-water interface. Surface supported lipid bilayers can also be formed by deposition of mixtures of lipids and detergents followed by detergent removal.

One example that represent a surface supported lipid bilayer that is complicated to generate, is a surface supported lipid bilayer that contains a large fraction (≥40 mole%) of cholesterol. For example, liposomes that contain a large fraction of cholesterol do not spontaneously form surface supported lipid bilayers upon adsorption on SiO₂. Other examples of lipid compositions that do not yield spontaneous surface supported lipid bilayer formation on SiO₂ surfaces upon liposome adsorption are significant fractions (>5 mole%) of lipids in the gel phase and, in general, protein-containing liposomes. Some such liposomes can be forced to form surface supported lipid bilayers using divalent ions like Ca²⁺, osmotic stress and raised temperature often in combination with very long (3-7 h) incubation times. However, there are many other lipid compositions for which surface supported lipid bilayer formation is still prevented. One example is the challenge of forming continuous surface supported lipid bilayer directly from cell membrane derived vesicles [Tanaka, M. et al, J. Am. Chem. Soc, 2004, (126) p3257] or the transfer of natural cell membrane components to surface supported lipid bilayers. This complication has, in turn, limited the applicability of surface supported lipid bilayers in, but not limited to, the fields of medical diagnostics, pharmaceutical development and cell membrane proteomics.

Therefore, in the assays used for forming surface supported lipid bilayers according to the state of the art, there is significant room for improvement regarding the complexity of the lipid composition in terms of number of different lipids, relative ratios of different lipids and number and ratios of other embedded biomolecules, such as, but not excluded to peptides, proteins and glycans.

### DEFINITIONS

Before the present method is described in detail, it is to be understood that this invention is not limited to the particular configurations, method steps, detection methods, transducing methods, sensors and materials disclosed herein as such configurations, method steps, detection methods, transducing methods, sensors and materials may vary. It is also to be understood that the terminology employed herein is used for the purpose of describing particular embodiments only and is not intended to be limiting since the scope of the present invention will be limited only by the appended claims and equivalents thereof.

It must also be noted that, as used in this specification and the appended claims, the singular forms "a", "an", and "the" include plural referents unless the context clearly dictates otherwise. Thus, for example, a reference to a surface supported lipid bilayer containing "an analyte" includes a mixture of two or more analytes, and furthermore, by "a first lipid bilayer" or "a second lipid bilayer" should, in the context of the present application, be understood as "one or more first lipid bilayers" and "one or more second lipid bilayers", respectively, and further examples which should be understood as "one or more" include "a spacer molecule", "a barrier", "a substrate", "a surface", among others.

The term "about" when used in the context of numeric values denotes an interval of accuracy, familiar and acceptable to a person skilled in the art. Said interval is preferably ± 10 %.

In describing and claiming the present invention, the following terminology will be used in accordance with the definitions set out herein.

The term "molecule" as used throughout the description and the claims denotes a group of at least two atoms held together by covalent bonds.

The term "molecular composition" as used throughout the description and the claims denotes a certain mixture of molecules where the molecules are present at a certain ratio.

The term "lipid" as used throughout the description and the claims denotes any fat-soluble molecules. Examples of lipids include but are not limited to fats, oils, waxes, cholesterol, sterols, monoglycerides, diglycerides, and phospholipids and co-block polymers.

The term "polypeptide" as used throughout the description and the claims denotes a single linear chain of amino acids, which build up proteins.

The term "oligosaccharide" as used throughout the description and the claims denotes a chain of a small number (typically three to ten) of sugars (monosaccharides).

The term "polysaccharide" as used throughout the description and the claims denotes a chain of several (typically more than ten) sugars (monosaccharides).

The term "naturally occurring molecules in the lipid bilayer" as used throughout the description and the claims denotes a molecule that originates from a natural cell membrane such as but not limited to lipids, carbohydrates, integral or peripheral membrane proteins.

The term "artificially provided molecules" as used throughout the description and the claims denotes a molecule that is synthetically produced or consist of a naturally occurring molecule in the lipid bilayer that is synthetically modified. Examples of artificially provided molecules include but are not limited to synthetically produced lipids, biotinylated lipids, or genetically modified proteins.

The term "lipid bilayer" as used throughout the description and the claims denotes a double layer structure of atoms or molecules and especially lipids. The term encompasses bilayers of all geometries including but not limited to planar and curved bilayers. Examples of bilayer structures include but are not limited to a bilayer structure in which at least one of the embedded molecules display mobility in a temperature interval.

The term "vesicle" as used throughout the description and the claims denotes a small (< 500 µm) closed cavity enclosing a liquid.

The term "micelle" as used throughout the description and the claims denotes an aggregate of surfactant molecules such as, but not limited to, lipids, sulfates, sulfonates and fatty alcohols, dispersed in a liquid colloid. A typical micelle in aqueous solution forms an aggregate with the hydrophilic region in contact with the surrounding solution and the hydrophobic regions in the micelle centre. Certain molecules forms inverted micelles.

The term "liposome" as used throughout the description and the claims denotes a vesicle comprising a lipid bilayer membrane. Liposomes comprise a liquid core. The lipid membrane of the liposome may comprise components such as but not limited to fats, oils, waxes, cholesterol, sterols, monoglycerides, diglycerides, phospholipids, glycolipids, steroids, co-block polymers, proteins, and other membrane-associated components.

The term "cell" as used throughout the description and the claims denotes the structural and functional unit of all living organisms such as but not limited to eukaryotic and prokaryotic cells.

The term "cell membrane derived liposome" as used throughout the description and the claims denotes a liposome where a fraction or the entire molecular composition originates from a cell membrane such as but not limited to the plasma membrane, the mitochondria and the endoplasmatic reticulum.

The term "native vesicles" as used throughout the description and the claims denotes a natural occurring liposome produced by the cell such as but not limited to synaptic vesicles.

The term "substrate" as used throughout the description and the claims denotes a solid material consisting of at least one type of surface material. The surface material consists of but are not limited to oxides, metals, semiconductors, polymers

The term "chemically modified surface" as used throughout the description and in the claims denotes a surface which has been chemically treated such that the surface characteristics have changed in terms of chemical properties and/or topography. The chemical modification can be performed but are not limited to by applying polymers, peptides, glycans, nucleic acids, affinity-tags on to the surface. The chemical modification also includes selective chemical deposition by for example chemical vapour deposition or treatment of the surface by, for example, etching or sputtering.

The term "mechanically modified surface" as used throughout the description and the claims denotes a surface which has been treated such that the surface topography has changed.

The term "homogeneous surface" as used throughout the description and in the claims denotes a surface which has no topographical structures larger than 10 nm and is composed of one kind of molecule, or one mixture of molecules.

The term "heterogeneous surface" as used throughout the description and the claims denotes a surface with topographical structure/s larger than 10 nm and/or is composed of more than one molecule or mixture of molecules.

The term "structure" as used throughout the description and the claims denotes a surface shape. A surface structure can be, but is not limited to, substantially planar, wells, pores and barriers.

The term "structural conformation" as used throughout the description and the claims denotes a shape of the lipid bilayer, which could be any shape from spherical to partially spherical, partially planar and planar or a combination of the above.

The term "surface supported lipid bilayer" as used throughout the description and claims denotes a lipid bilayer that is supported on a substrate.

The term "surface supported liposome" as used throughout the description and claims denotes a liposome supported on substrate. The surface supported liposome is a substantially spherical lipid bilayer.

The term "substantially planar surface supported lipid bilayer" as used throughout the description and claims denotes a surface supported lipid bilayer with an edge. The "substantially planar surface supported lipid bilayer" is a continuous lipid bilayer sheet which does not enclose a liquid by itself. The "substantially planar surface supported lipid bilayer" may be continuous over an area range of, for example but not limited to, at least 100 µm². The "substantially planar surface supported lipid bilayer" may have curvature such as but not limited to a "substantially planar surface supported lipid bilayer" following the topography of the surface. The same shall apply to the term "substantially planar".

The term "supported lipid bilayer edge" as used throughout the descriptions and the claims denotes the edge of a substantially planar surface supported lipid bilayer.

The terms "fusion", "fusing" and "fuse" as used throughout the descriptions and the claims denotes the merger of two lipid bilayer structures and mixing of their components. In the specific case of a substantially planar surface supported lipid bilayer and a surface supported liposome, the liposome will be transformed into a part of the substantially planar supported lipid bilayer upon fusion.

The term "membrane" as used throughout the description and the claims comprises all types of membrane such as but not limited to a bilayer membrane. A membrane may comprise molecules such as but not limited to proteins and lipids.

The term "membrane-associated molecule" as used throughout the description and the claims denotes molecules that are associated to a lipid bilayer by interactions including but not restricted to hydrophobic, charge, covalent interactions and interactions based on specific recognition between the membrane-associated molecules and molecules in the lipid bilayer, and combinations of said interactions. The membrane-associated molecules can be situated on top of the upper bilayer leaflet and can be partially embedded in the upper bilayer leaflet, in both bilayer leaflets or spanning the lipid bilayer. Examples of membrane-associated molecules include but are not limited to membrane-associated proteins such as insulin receptors, integrins, cadherins, selectins, receptor proteins, glycoporins, rhodopsins, cholera toxin, glycoproteins, G proteins, proton pumps, peripheral enzymes, hormones, toxins, inhibitors and antimicrobial peptides, lipids and carbohydrates.

The term "means for anchoring a molecule" as used throughout the description and the claims denotes means by which a molecule can be anchored to a lipid bilayer including but not restricted to hydrophobic, charge, covalent interactions and interactions based on specific recognition between the molecule to be anchored and the molecules in the lipid bilayer and combinations of said interactions. The interactions are such that the anchored molecule is situated on top of the upper bilayer leaflet, in the upper bilayer leaflet, in both bilayer leafletsor spanning the lipid bilayer.

The term "tether" as used throughout the description and the claims denotes the attachment or entrapment of a material to a surface in a manner that confines, but not necessarily restricts the movement of the material.

The term "spacer molecule" as used throughout the description and the claims denotes a molecule or a mixture of molecules attached between the lipid bilayer and the support. A spacer molecule is at least one selected from the group consisting of but not limited to a polymer, peptide, glycan, nucleic acid, an affinity tag.

The term "barrier" as used throughout the description and the claims denotes a topographical, chemical or molecular feature which hinders the motion of the substantially planar surface supported lipid bilayer in a certain direction.

The term "immobilize" as used throughout the description and the claims denotes hindering a particle to move in at least one dimension.

The term "sensor" as used throughout the description and the claims denotes a device that measures a physical quantity and converts it into a signal, which can be read by an observer or an instrument. A "sensor" can be based on techniques such as, but not limited to, techniques sensitive to interfacial refractive index contrasts, including surface plasmon resonance and ellipsometry, gravimetric techniques, including quartz crystal microbalance devices and surface acoustic wave devices, electrochemical, patch clamp or electrical impedance spectroscopy devices, scanning probe techniques, mass spectrometry techniques, fluorescence readout principles and techniques based on optical interference including dual polarization interferometry.

The term "hydrodynamic flow" as used throughout the description and the claims denotes the motion of a liquid.

The term "electroosmotic flow" as used throughout the description and the claims denotes the motion of liquid caused by ions moving in the liquid due to an applied potential across a fluid conduit.

The term "self-spreading mechanism" as used throughout the description and the claims denotes the mechanism by which a substantially planar supported lipid bilayer spreads out over hydrophilic substrates or a substantially planar supported lipid monolayer spreads out over a hydrophobic substrate in the presence of a lipid material reservoir and a water-based liquid.

The term "flow path" as used throughout the description and the claims denotes a path wherein a flow of liquid can be applied. A "flow path" can be, but is not limited to, a microfluidic channel, nano-channel or a capillary.

The term "microfluidic channel" as used throughout the description and the claims denotes a channel of which at least one dimension of the channel (length, width or height) is in the range of 1 mm - 1 µm.

The term "nano-channel" as used throughout the description and the claims denotes a channel where at least one dimension of the channel is on the scale of 1 µm - 1 nm.

The term "capillary" as used throughout the description and the claims denotes a narrow tube with a radius so small and a surface tension so strong that a liquid will move along the tube.

The term "pore" as used throughout the description and the claims denotes a hole in the substrate going through the substrate, making it possible to let a liquid flow through the substrate.

The term "well" as used throughout the description and in the claims denotes a cavity in the substrate that does not go through the substrate.

### SUMMARY OF THE INVENTION

In view of the above-mentioned and other drawbacks of the prior art, a general object of the present invention is to overcome the above-mentioned problem and to provide a method for fusing lipid bilayers.

According to a first aspect of the present invention, these and other aspects are achieved through a method for fusing lipid bilayers comprising the steps of: providing a supportive substrate having a surface, providing a first lipid bilayer on the surface of the supportive substrate, providing a second lipid bilayer on the surface of the supportive substrate; and providing a force on at least the first lipid bilayer such that the first lipid bilayer is driven along the surface of the substrate towards the second lipid bilayer and the first lipid bilayer is fused with the second lipid bilayer.

By "a first lipid bilayer" and "a second lipid bilayer" should, in the context of the present invention, be understood as "one or more first lipid bilayers" and "one or more second lipid bilayers", respectively. Thus, the method according to the invention include embodiments having an arbitrary numbers of first and second lipid bilayers on the surface of the substrate.

According to one embodiment of the invention, the force may be provided by a flow of a liquid along the first lipid bilayer.

Thereby, a hydrodynamic force may be provided on the lipid bilayer causing the lipid bilayer to move in the direction of the flowing liquid. The flow can for example be pressure driven or due to electroosmosis or any combination thereof.

According to one embodiment of the invention, the force is provided by a hydrodynamic flow or an electroosmotic flow, or by any combinations thereof.

Furthermore, in addition to the above-mentioned force due to flow of a liquid, an internal force due to a self-spreading mechanism may also be present affecting the motion of the lipid bilayer.

According to one embodiment of the invention, the first lipid bilayer may be different from the second lipid bilayer with respect to at least one of structural conformation and molecular composition.

The molecular composition of, for example, the second lipid bilayer may be configured such that a stronger interaction between the second lipid bilayer and the surface of the supportive substrate is achieved, compared to the corresponding interaction between the first lipid bilayer and the surface of the supportive substrate. Thus, the first lipid bilayer may be more mobile on the surface of the substrate, than the second lipid bilayer. Consequently, despite the fact that the provided force may act on both the first lipid bilayer and the second lipid bilayer, the first lipid bilayer can be moved to the less mobile second lipid bilayer such that the first lipid bilayer and second lipid bilayer are fused. Similarly, by configuring the structural conformations of the first and second lipid bilayer such that the second lipid bilayer is less mobile on the surface of the substrate, compared to the first lipid bilayer, a fusion of the first lipid bilayer and the second lipid bilayer, according to the above-discussed method, can be achieved.

According to one embodiment of the invention, the structural conformation of the first bilayer may be substantially planar.

Thereby, the lipid bilayer may be driven along the surface of the substrate in a rolling motion, where the upper and lower leaflets of the lipid bilayer may have different drift velocities.

According to one embodiment of the invention, the second lipid bilayer may be a vesicle.

It has been found that lipid vesicles adsorbed to the surface may remain essentially stationary on the surface even though a substantially planar lipid bilayer can be moved by the provided force. Thus, if the second lipid bilayer compriseslipid vesicles these will not move and can be fused with the first, mobile, lipid bilayer. Thereby, this method may be suitable for fusing a vesicle with, for example, a substantially planar lipid bilayer. In particular, as the vesicle may be a cell membrane including naturally occurring membrane-bound molecules, fusing such vesicle with, for example, a substantially planar lipid bilayer affords a new fused lipid bilayer having a substantially planar structural conformation and including the naturally occurring membrane-bound molecules, thus the method according to the invention facilitates the study of these membrane-bound molecules. In one embodiment, the lipid bilayers may be fused with micelles, according to the above-discussed method.

According to one embodiment of the invention, the substrate may advantageously comprise a flow path. The flow path may, for example, be a microfluidic channel, a nanofluidic channel or a glass capillary, and furthermore, such flow path may be embedded in the substrate. A lipid bilayer may be provided and driven, according to the above-discussed method, on any walls of the channel.

According to one embodiment of the invention, the substrate may be made from one or more materials.

The materials of the substrate may be chosen such that one surface of the substrate may have different surface properties compared to a neighboring surface of the substrate. Thus, by using different materials with different surface properties, the interactions between the surface of the substrate and the lipid bilayer may be adapted to be different on different parts of the surface. For example, materials may be chosen such that the substrate comprises a portion with a hydrophobic surface and a portion with a hydrophilic surface and thus a lipid bilayer will have different structural conformation and its molecular constituents may have different mobility, due to different surface interactions, along these two portions of the substrate.

According to one embodiment of the invention, the surface of the substrate may at least partially be chemically or mechanically modified, in order to adapt interactions between the lipid bilayer and the surface of the substrate.

Thereby, as discussed above, by modifying the surface properties, the interactions between the surface of the substrate and the first lipid bilayer and the interactions between the surface of the substrate and second lipid bilayer may be adapted to be different, thus enabling fusion of the first lipid bilayer and the second lipid bilayer according to the above-discussed method. For example, the surface of the substrate may be chemically modified to have hydrophobic properties by providing a monolayer of lipids on a portion of the surface. Furthermore, by adapting the topography of the structure of the surface, the transport of lipid bilayers along the surface may be controlled. The surface of the substrate may, for example, have a smooth topography or a rough topography or a structured topography, or alternatively, the surface may comprise different combinations of smooth portions, rough portions and structured portions.

Furthermore, the topography of the surface may be such that wells or pores are provided on a portion of the surface of the substrate. The wells or pores can be configured to accommodate lipid bilayers such as, for example, vesicles. Thus, vesicles may be positioned on the portion of the surface with wells or pores. Therefore, a substantially planar surface supported lipid bilayer may be provided on an adjacent portion of the surface with no wells or pores, and driven towards, and fused with the positioned lipid bilayers. Thereby, a space between the resulting fused lipid bilayers on the portion of the surface with wells or pores, and the surface of the substrate may be provided. Such space may be desirable, for example, in order to avoid denaturation of lipid-bound proteins due to their interactions with the surface of the substrate. Such space may also be desirable for investigations of molecular transport across the lipid bilayer.

According to one embodiment of the present invention, the surface of said substrate may comprise an embedded sensor element.

Modification of the surface of the substrate may comprise providing an embedded sensor element for measuring, for example, binding of molecules to membrane-associated molecules in the lipid bilayer or transportation of molecules across the lipid bilayer. If the sensor element is designed to probe reactions at or close to the wells the molecular constituents of vesicles immobilized at or close to the pores will dominate the measured response.

According to one embodiment of the invention, the surface of the substrate may comprise a spacer molecule.

According to one embodiment of the invention, the spacer molecule may be positioned between the lipid bilayer and the surface of the substrate, thus creating a space which may be filled with, for example, water molecules.

This may, as discussed above, be desirable in order to avoid denaturation of lipid-bound proteins due to their interactions with the surface of the substrate. This may also, as discussed above, be desirable for investigations of molecular transport across the lipid bilayer. The chemical interactions between the lipid bilayer and the spacer molecule may, however, be strong such that the mobility of the lipid bilayer may be reduced. Hence, a spacer molecule may hold a lipid bilayer at a fixed position on the surface of the substrate as another lipid bilayer is driven towards and fused with the lipid bilayer bound to the spacer molecule, according to the method described above. The resulting fused lipid bilayer may also be bound to the spacer molecule. Furthermore, by using a spacer molecule, identical lipid bilayer may be fused. The spacer molecule may also be positioned selectively in or close to wells or pores in the substrate.

According to one embodiment of the present invention, the surface of the substrate may comprise a barrier.

A "barrier" should, in the context of the present application, be understood as any structural hinder on the surface of the substrate, wherein such hinder may be provided by chemically or mechanically modifying the surface of the substrate. The barrier may be provided within the substrate, for example, as a wall in a flow channel, or by applying other materials, such as proteins or polymers, to the surface of the substrate.

By providing a barrier on the surface the movement and confinement of a lipid bilayer on the surface may be controlled.

According to an another aspect of the present invention, there is a method for transporting membrane-associated molecules on a lipid bilayer such that the membrane-associated molecules are accumulated, the method comprising the steps of: providing a supportive substrate having a surface; providing a lipid bilayer, wherein the lipid bilayer comprises membrane-associated molecules; and providing a force on the lipid bilayer such that the membrane-associated molecules are transported in a first direction, and accumulated at a front edge of said lipid bilayer.

The membrane-associated molecules may be substantially embedded in the lipid bilayer, however, some portion of the membrane-associated molecules may be protruding from the surface of the lipid bilayer. Consequently, the force provided on the lipid bilayer also acts on the protruding parts of the membrane-associated molecules such that they may be moved along the lipid bilayer. The effective shear force is generally stronger on the protruding part of the membrane-associated molecules compared to the shear force acting on the surface of lipid bilayer, thus the a membrane-associated molecules may have higher drift velocity than the front of the lipid bilayer. It should be noted that transportation of membrane-associated molecules through the lipid bilayer does not necessarily require a moving lipid bilayer as is discussed further below.

Accumulation of membrane-associated molecules at a front edge may be advantageous in order to achieve a subsequent separation as discussed below.

According to another embodiment of the present invention, the above-described accumulation of the membrane-associated molecules may be followed by a separation step, comprising: providing a force on the lipid bilayer such that the membrane-associated molecules are transported in a second direction, wherein the second direction is different from, preferably opposite to, the first direction, separating the membrane-associated molecules.

By reversing the direction of the bulk flow the accumulated membrane-associated molecules may move away from the edge of the bilayer. In this scenario, each type of membrane-associated molecule, for example a protein, can be expected to have a characteristic drift velocity determined by the size and conformation of the membrane-associated molecule, as well as the frictional coupling between the membrane-associated molecule and the lipid bilayer. A large membrane-associated molecule that protrudes significantly into the bulk solution is expected to have a higher drift velocity than a small, flat membrane-associated molecule remaining close to the substantially planar surface supported lipid bilayer, as the large membrane-associated molecule should experience a higher hydrodynamic force from the flowing liquid. In addition, an membrane-associated molecule with many anchors to the substantially planar surface supported lipid bilayer is expected to have a lower drift velocity than a similarly sized membrane-associated molecule with fewer anchors to the substantially planar surface supported lipid bilayer, as the membrane-associated molecule with many anchors experiences a stronger frictional drag from the lipid bilayer. Due to the different drift velocities the membrane-associated molecules may separate into spatially separated bands in the substantially planar surface supported lipid bilayer. It should be noted that transportation of molecular constituents of a lipid bilayer along the surface of the substrate does not necessarily require a moving lipid bilayer as is discussed further below.

According to one embodiment of the present invention, the separation may be enhanced by selective binding of a bulky molecule or particle to the membrane-associated molecules.

Thereby, a larger hydrodynamic force may be provided to the membrane-associated molecules as the bound molecule or particle protrudes significantly into the bulk solution, which results in a higher drift velocity as compared to the membrane-associated molecule alone as discussed above.

According to one embodiment of the present invention, the force may be provided by providing a flow of a liquid along the first lipid bilayer.

Thereby, a hydrodynamic force may be provided on the lipid bilayer causing the membrane-associated molecules to move along the lipid bilayer in the direction of the flowing liquid. The flow can for example be pressure driven or due to electro-osmosis or any combination thereof.

According to one embodiment of the present invention, the force may be provided such that the lipid bilayer is driven along the surface of the substrate.

Thereby, in addition to moving the membrane-associated molecule along the lipid bilayer, the provided force may also cause the lipid bilayer to be driven along the surface of the substrate.

According to one embodiment of the present invention, the structural conformation of the lipid bilayer may advantageously be substantially planar.

A substantially planar structural conformation gives wide distribution of the lipid bilayer which may be desirable to enable the accumulation and separation of membrane-associated molecules according to the above described method.

According to one embodiment of the present invention, the surface of the substrate may comprise a barrier.

By introducing a barrier on a surface of the substrate, as discussed above, the movement of a lipid bilayer on the surface may be controlled. A barrier may be used to stop and accumulate force-driven lipid bilayers at the position of the barrier, alternatively the lipid bilayer may be provided adjacent to the barrier. Subsequently, by providing a force on the lipid bilayer, the membrane-associated molecules are transported through the lipid membrane towards the edge of the lipid bilayer adjacent to the barrier. Thereby, accumulation of the membrane-associated molecules may be achieve at a fixed known position on the surface of the substrate, facilitating, for example, the analysis of the accumulated membrane-associated molecules. Furthermore, by designing the shape of the barrier, the shape of the front edge of the lipid bilayer may be adapted. The shape of the lipid bilayer may be an important factor for achieving an efficient separation of the membrane-associated molecules according to the method described above.

According to one embodiment of the present invention, the surface of the substrate may comprise a spacer molecule.

The spacer molecule may be attached to or associated with the surface of the substrate and the lipid bilayer, thus providing a linkage and a space between the surface and the lipid bilayer. The interaction between the spacer molecule and the lipid bilayer may be adapted such that a fraction of molecules in the lipid bilayer can be held fixated at the position of the spacer molecule on the surface of the substrate.

According to one embodiment of the present invention, the membrane-associated molecules may comprise at least one of proteins, oligosaccharides, nucleotides, lipids, polymers of biological relevance, or any combinations thereof.

According to one embodiment of the present invention, the membrane-associated molecules may comprise at least one of naturally occurring molecules in the lipid bilayer or artificially provided molecules.

Thereby, the method according to the invention facilitates the separation and study a mixture of naturally occurring membrane-bound molecules, such as for example, membrane-bound proteins or glycolipids. The method according to the invention may also be used to separate a mixture of artificially provided molecules in the lipid bilayer According to one embodiment of the present invention, the lipid bilayer may have means for anchoring the membrane-associated molecules

According to one embodiment of the present invention, the membrane-associated molecules may be membrane-associated to the lipid bilayer by the means for anchoring.

### BRIEF DESCRIPTION OF THE DRAWINGS

This and other aspects of the present invention will now be described in more detail, with reference to the appended drawings showing embodiment(s) of the invention, wherein
FIGURE 1 shows a method of producing a planar surface supported lipid bilayer according to the present invention;
FIGURE 2 shows a planar surface supported lipid bilayer in a microfluidic channel;
FIGURE 3 shows a planar surface supported lipid bilayer in a microfluidic channel in which the part of the channel not covered by the planar supported lipid bilayer is covered with liposomes with a chemical composition that differs from the planar surface supported lipid bilayer;
FIGURE 4 shows a micrograph of a planar surface supported lipid bilayer and liposomes in a microfluidic channel;
FIGURE 5 shows a micrograph of the planar surface supported lipid bilayer after incorporation of material as the liposomes have fused with the planar surface supported lipid bilayer upon movement of the latter;
FIGURE 6 show intensity profiles as a function of time from two different fluorescent dyes incorporated in the planar surface supported lipid bilayer and the liposomes respectively;
FIGURE 7 shows the result from a diffusivity analysis in planar supported lipid bilayer with different molecular compositions;
FIGURE 8 shows intensity profiles from fluorescent cholera toxin bound to the planar supported lipid bilayer;
FIGURE 9 shows micrographs of two populations of fluorescent cholera toxin bound to the planar supported lipid bilayer;
FIGURE 10 shows intensity profiles as a function of time of two populations of fluorescent cholera toxin bound to the planar supported lipid bilayer;
FIGURE 11 shows micrographs of two populations of fluorescent cholera toxin and one population of streptavidin bound to the planar supported lipid bilayer; and
FIGURE 12 shows micrographs of one planar supported lipid bilayer in the liquid phase and one planar supported lipid bilayer in the gel phase.

### DETAILED DESCRIPTION

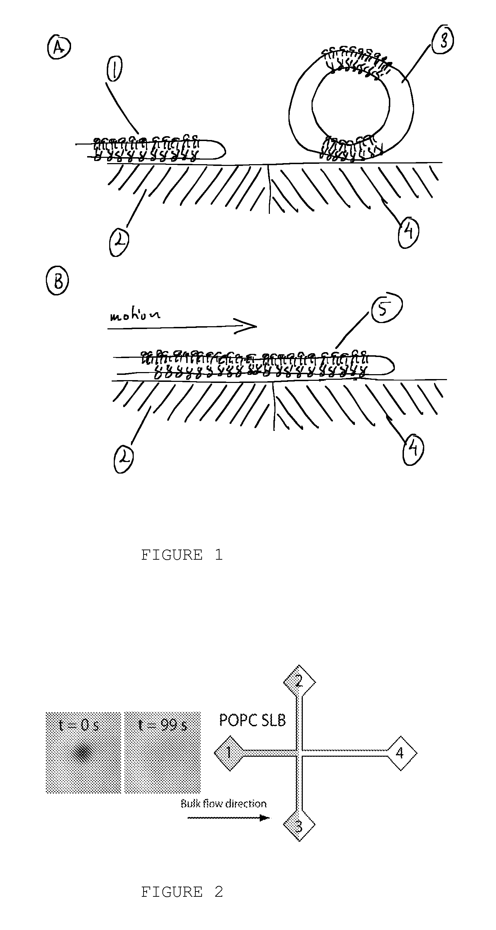

The present invention is further described by the following detailed description. A method of producing a planar surface supported lipid bilayer according to the present invention is shown in Figure 1. According to one aspect of the invention a substantially planar surface supported lipid bilayer (1) is formed on a substrate (2). Liposomes (3) are deposited on a substrate (4). Figure 1B shows a substantially planar surface supported lipid bilayer (5) on substrate (4) formed by moving the substantially planar surface supported lipid bilayer (1) in the direction towards the surface supported liposomes (3). Upon interaction, the surface supported liposomes (3) fuse with the substantially planar surface supported lipid bilayer and the material of the surface supported liposomes is incorporated into the substantially planar surface supported lipid bilayer. Substrates (2) and (4) can, but must not, be the same material. The surface supported lipid bilayers can, but must not have, the same molecular composition.

Below, the invention is further described by the four specific embodiments. However, although the invention is described with regard to its preferred embodiments which comprise the best mode presently known to the inventors it should be understood that various changes and modifications as would be obvious to one having the ordinary skill in this art may be made without departing from the scope of the invention as set forth in the claims appended hereto.

### Formation of a substantially planar surface supported lipid bilayer from liposomes which do not spontaneously form substantially planar surface supported lipid bilayers upon liposome adsorption.

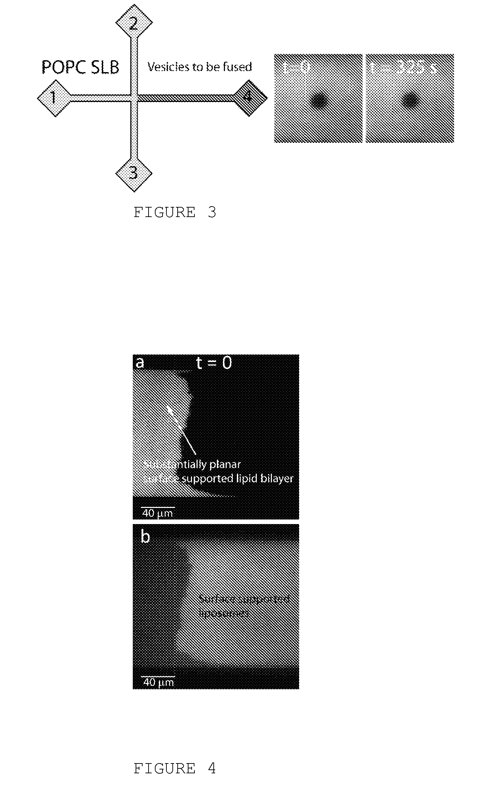

To illustrate that the method may be used to enable formation of a substantially planar surface supported lipid bilayer with a lipid composition substantially determined by the lipid composition of the surface-adsorbed liposomes, a 1-Palmitoyl-2-Oleoyl-sn-Glycero-3-Phophocholine (POPC) substantially planar surface supported lipid bilayer front was driven towards adsorbed liposomes composed of (1) 1,2-dioleoyl-sn-glycero-3-phosphocholine (DOPC) and cholesterol at a 60:40 mole% ratio, **(2)** DOPC and cholesterol at a 50:50 mole% ratio and **(3)** DOPC, 1,2-dioleoyl-sn-glycero-3-phosphoethanolamine (DOPE) and cholesterol at a 39:21:40 mole% ratio. All three compositions are shown not to spontaneously form substantially planar surface supported lipid bilayer upon liposome adsorption. This is illustrated for composition (**1**) in Figure 3, showing the absence of recovery on micron length scales.

Figure 2 shows a microfluidic cross-channel (150 µm wide and 110 µm high) created by bonding a PDMS replica to a supporting glass slide. The numbers (1-4) indicates the indexing of the channel arms in the cross channel.

A first substantially planar surface supported lipid bilayer was formed in the left part of the channel, by flowing (10 µl/min) a POPC liposome suspension (0.1 mg/mL) labelled with 1 wt% 2(12-(7-nitrobenz-2-oxa-1,3-diazol-4-yl)amino)dodecanoyl-1-hexadecanoyl-sn-glycero-3-phosphocholine (NBD C₁₂-HPC) (Figure 2, light grey) from channel arm 1 to channel arms 2 and 3, and simultaneously having a flow of pure buffer (20 µl/min) from channel arm 4 to channel arms 2 and 3. The first substantially planar surface supported lipid bilayer showed full recovery (Figure 2) with a diffusivity of around 2.2 µm²s-¹ and an immobile fraction of < 1%, according to a fluorescence recovery after photobleaching (FRAP) analysis as described in [Jönsson, P. et al, Biophys. J. 2008, (95) p5334].

Figure 3 shows a first substantially planar surface supported lipid bilayer driven forward by the shear force from a bulk flow (250 µl/min) which results in a speed of the surface supported lipid bilayer edge of ~0.2 µm/s in the direction from channel arm 1 to channel arm 4, while channel arms 2 and 3 were kept closed. The exerted force per surface area was then approximately 20 Pa. The Lissamine™ rhodamineB 1,2-dihexadecanoyl-sn-glycero-3-phosphoethanolamine (rhodamine-DHPE)labelled (1 wt%) surface supported liposomes (Figure 3, dark grey) of composition (**1**) were subsequently adsorbed in front of the first substantially planar supported lipid bilayer, by flowing (20 µl/min) a liposome suspension (0.1 mg/ml) from channel arm 1 to channel arm 4, while keeping channel arms 2 and 3 closed. No spontaneous supported lipid bilayer formation was observed for liposomes of compositions (**1**), (**2**) and (**3**). This is illustrated using FRAP (Figure 3) for adsorbed liposomes of composition (1) in which essentially no recovery of the bleached spot was observed.

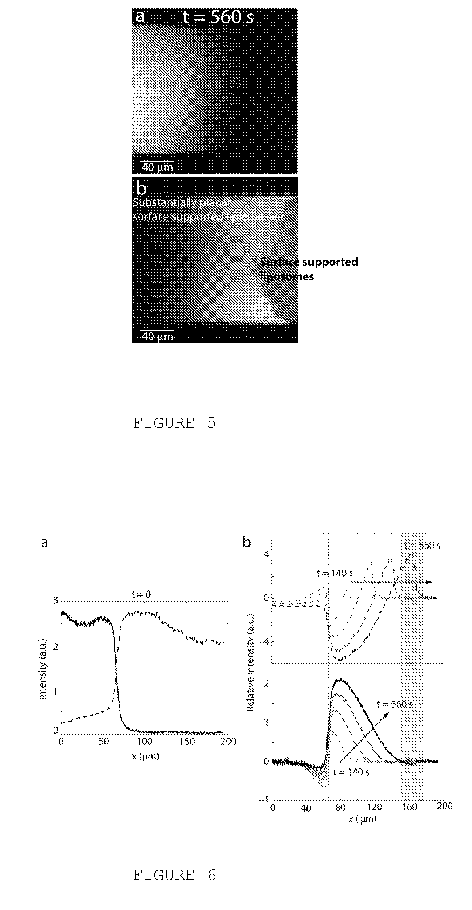

In the next step, the bulk flow was again increased to 250 µl/min, which resulted in a constant movement of the surface supported lipid bilayer edge with a speed of ~0.2 µm/s, while the surface supported liposomes remained stationary. The interaction between the moving NBD-labeled bilayer front and non-bilayer forming, rhodamine-labeled liposomes of composition (**1**) was followed in real time (Figures. 4, 5 and 6) using total internal fluorescence (TIRF) microscopy (inverted, Nikon Eclipse Ti-E), by alternating between FITC (Figures. 4A and 5A) and TRITC (Figures. 4B and 5B) filter cubes.

Micrograph snapshots were taken before (Figure 4) and 560 sec after initiation of the motion (Figure 5) of the substantially planar surface supported lipid bilayer. Figure 5A shows the first substantially planar surface supported lipid bilayer that has been moved using a hydrodynamic flow against the surface supported liposomes which have fused and incorporated its material into the first substantially planar surface supported lipid bilayer

From the micrographs in Figures 4 and 5 it is seen that the intensity of the NBD lipids, which were initially present in the surface supported lipid bilayer only, gradually decreases from left to right. In contrast, the intensity of the rhodamine-labeled lipids, which were initially present in the surface supported liposomes only, gradually increases from left to right, reaching a value close to the edge that is larger than that of the rhodamine labelled surface supported liposomes.

Although these features alone illustrates that the moving surface supported lipid bilayer front has fused with the adsorbed liposomes, a more detailed inspection is possible from the time evolution of the distance dependent intensity profiles of NBD (solid line) and rhodamine (dashed line), as shown in Figure 6.

An analysis of the time resolved microscopy analysis reveals that the cholesterol-containing liposomes (Figures. 4 and 5) fuse with the supported lipid bilayer (Figure 4) to form a supported lipid bilayer structure containing cholesterol and rhodamine-DHPE (Figure 5). Figure 6 shows intensity profiles along the microfluidic channel, corresponding to the two different dyes used (rhodamine = dashed and NBD = solid line) for different times. At t = 0 (Figure 6a) the intensity profiles show the sharp edge between the NBD-labeled first substantially planar surface supported lipid bilayer (solid line) and the rhodamine-labeled cholesterol liposomes (dashed line) prior to initiation of the motion of the supported lipid bilayer structure. As time evolves after onset of the supported lipid bilayer motion (in steps of 140 s in Figure 6b, the rhodamine-labeled cholesterol-containing liposomes fuse with the NBD-labeled POPC first substantially planar surface supported lipid bilayer and hence add rhodamine-labeled lipids to a second substantially planar surface supported lipid bilayer. This is reflected as a positive build up in the dashed relative intensity (*I* = *I*(*t*)-*I*(*t*=0)) profiles versus time (Figure 6b).

At t = 0 (Figures 4 and 6a) the two intensity profiles display a ~10 **µ**m sharp edge between the two intensity profiles, with only minor overlap, attributed to fusion of rhodamine-labeled liposomes at the edge of the first surface supported lipid bilayer. This leads to limited delivery of rhodamine-labeled lipids to the NBD-labeled bilayer already before motion of the surface supported lipid bilayer is initiated. As the substantially planar surface supported lipid bilayer edge is driven forward (Figure 6b), the relative intensity profiles for rhodamine-DHPE (dashed line, Figure 6b) show a decrease to the left of the position of the substantially planar surface supported lipid bilayer edge at t = 0 (indicated by a dotted line in Figure 6b). To the right of the position of the surface supported lipid bilayer edge at t = 0 (dotted line, Figure 6b), there is a decrease for around 10 **µ**m followed by an increase in the relative intensity profiles for rhodamine-DHPE. The decrease is attributed to rhodamine-DHPE molecules in the substantially planar surface supported lipid bilayer that move towards the front (right direction), which leads to depletion of rhodamine-DHPE in the left regions of the channel. This interpretation is supported by the increase in the relative intensity observed for the NBD C₁₂-HPC molecules (solid lines in Figure 6b). The observed overlap of the relative intensity profiles for rhodamine-DHPE and NBD C₁₂-HPC demonstrates mixing of the two dyes, which is in agreement with formation of a substantially planar surface supported lipid bilayer with a composition that is a mixture of POPC and (**1**). At least three additional observations support this interpretation. First, the relative intensity profile of rhodamine-DHPE, which is known to be preferentially located in the upper leaflet of the substantially planar surface supported lipid bilayer, displays a maximum positioned **µ**10 µm to the left of the sharp decrease in the intensity. This type of accumulation of rhodamine-DHPE was previously observed to occur at the front of a moving surface supported lipid bilayer made from POPC [Jönsson, P. et al, J. Am. Chem. Soc, 2009, (131) p5294], and was in this case attributed to the bulky, and negatively charged, head group of rhodamine-DHPE which forces the bilayer edge to act as a molecular sieve for the rhodamin-DHPE lipids. Second, at longer times, the gradient of the NBD profile extends further into the region that was originally modified with surface supported liposomes, demonstrating that the NBD C₁₂-HPC lipid bilayer has been moved forward in the channel. Third, upon interruption of the flow, the two differently labelled lipids became homogeneously mixed at a time scale of ~60 minutes, which is good agreement with the expectation for lipids with diffusivities of around 2µm²s⁻¹ Similar results to those shown in Figures 4, 5 and 6 were obtained for compositions (**2**) and (**3**).

After 560 s there is also a region to the right (marked in gray in Figure 5b) which contains no NBD-labeled lipids but where the accumulation of rhodamine-DHPE molecules is significant. Hence, under the reasonable assumption that the diffusion of the NBD C₁₂-HPC is not significantly slower than that of POPC [Jönsson, P. et al, J. Am. Chem. Soc, 2009, (131) p5294], the composition of the newly generated surface supported lipid bilayer may in this region contain a significant fraction of lipid composition (**1**). This signature also suggests that the build up of lipid material from the surface supported liposomes to the front of the surface supported lipid bilayer was, at this flow speed, faster than the backward diffusion of lipid material. This thus demonstrates an efficient means to generate a substantially planar surface supported lipid bilayer with a composition substantially defined from liposomes that do not spontaneously form substantially planar surface supported lipid bilayer.

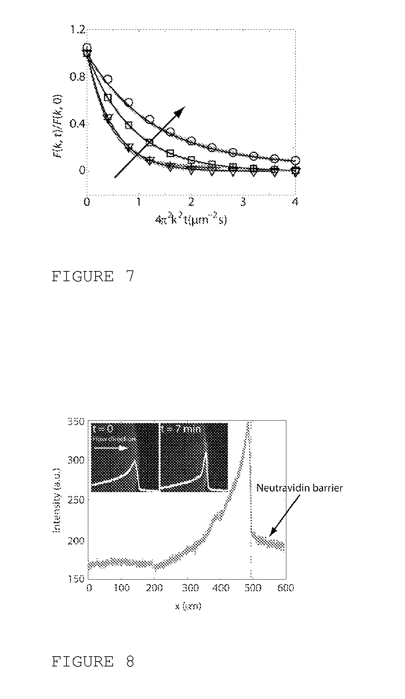

Fluorescence recovery after photobleaching (FRAP) analysis was made to extract the diffusivity of rhodamine-DHPE in the second substantially planar surface supported lipid bilayer made from a layer of adsorbed liposomes containing (**1**) DOPC:CH (40:60 mole%), (**2**) DOPC:CH (50:50 mole%) and (**3**) DOPC:DOPE:CH (39:21:40 mole%), rendering diffusivities of 1.97±0.11 µm²s⁻¹, 1.23±0.11 µm²s⁻¹ and 0.78 µm²s⁻¹, respectively. The diffusivities for the different lipid compositions are shown in Figure 7. In this example, the substantially planar surface supported lipid bilayer made from POPC was removed from channel arms 1, 2 and 3 using SDS, to minimize slow lipid mixing.

### Formation of substantially planar surface supported lipid bilayers from liposomes derived from native cell membranes

To explore an additional potential of the method, we investigated the possibility to fuse the moving edge of a first substantially planar surface supported lipid bilayer with surface supported cell membrane-derived liposomes. The procedure described above for a substantially planar surface supported lipid bilayer made from POPC and surface supported liposomes of compositions (**1**), (**2**) and (**3**) was repeated, with the exceptions that (i) the surface supported liposomes were replaced for surface supported cell-membrane-derived liposomes and (ii) the NBD-labeled substantially planar surface supported lipid bilayer made from POPC was replaced for an unlabeled substantially planar surface supported lipid bilayer made from POPC.

In order to verify transfer of native cell membrane constituents, cholera toxin B subunit-fluorescein conjugated (CTB-FITC) was used to identify the presence of monosialoganglioside G_{M1} (GM1) lipids, which are known to be present in the outer membrane of 3T3 fibroblast cells. The unlabeled first substantially planar surface supported lipid bilayer was fused with surface supported cell membrane-derived liposomes as described above. Thereafter, the motion of the substantially planar surface supported lipid bilayer was interrupted, and NeutrAvidin (20 µg/ml) was adsorbed (for ~10 min) in front of the substantially planar surface supported lipid bilayer. Besides acting as a molecular barrier for further movement of the surface supported lipid bilayer, the adsorbed layer of NeutrAvidin also helped prevent unspecific adsorption of CTB-FITC to the substrate in front of the substantially planar surface supported lipid bilayer front, as illustrated in Figure 8.

Upon addition of CTB-FITC, preferential binding occurred at the front of the substantially planar surface supported lipid bilayer, as shown in Figure 8 with the front indicated with a dashed line. This thus demonstrates that the GM1 lipids have been preferentially accumulated at the front of the bilayer. This is attributed to the larger hydrodynamic force exerted on the GM1 moiety compared to that of the POPC lipid. Accumulation of GM1 at the front of the bilayer is therefore due to a faster motion of GM1 lipids than the majority of the molecules in the rest of the surface supported lipid bilayer.

By increasing the flow rate to 250 µl/min after completed binding of CTB-FITC, it was also possible to further enrich the GM1-CTB-FITC complex at the front of the substantially planar surface supported lipid bilayer (inset micrographs at t=0 and t=7 min in Figure 8). This shows that the lateral mobility of the GM1-CTB-FITC complex was sustained in the substantially planar surface supported lipid bilayer, and illustrates a new means to locally enrich low abundant components derived from cell membranes.

### Separation of proteins bound to a substantially planar surface supported lipid bilayer

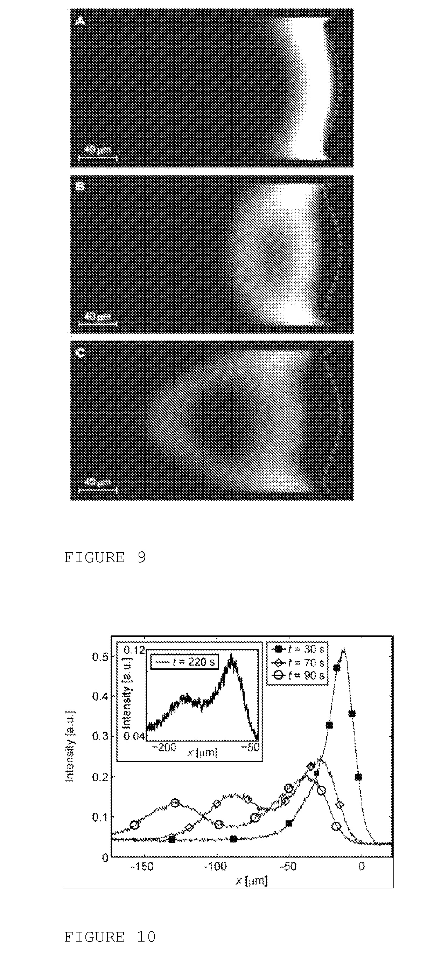

We illustrate that the method can be used not only to accumulate but also to separate different populations of molecules associated with the substantially planar surface supported lipid bilayer. A first example demonstrates the separation of two populations of the same protein. A surface supported lipid bilayer, containing a small fraction of GM1 lipids was formed in a microfluidic channel. The protein CTB-FITC, which binds specifically to the GM1-lipids (see above), was subsequently added to the channel. When reversing the flow, after first having accumulated CTB-FITC at the lipid bilayer edge of the substantially planar surface supported lipid bilayer, the intensity peak was observed to divide into multiple peaks, with different drift velocities (Figures 9 and 10). Figure 9 shows fluorescence micrographs of two populations of CTB-FITC generated by reversing the direction of the flow after flow-induced accumulation of CTB-FITC. The dotted line in Figure 9 shows the front of the bilayer at t = 0. The flow rate was 200 **µ**L/min and the time after flow reversal for the different figures are (a) 30 s, (b) 70 s and (c) 110 s. Figure 10 show line profiles of the intensity of cholera toxin at the centre of the channel at different times after a 200 µL/min bulk flow has been applied in the negative x-direction (from right to left in Figure 9) at t=0 sec. The intensities are normalized to the peak intensity at t=0 sec. The peak was observed to divide into two mobile fractions relatively quickly, whereas a third fraction is observed to separate from the slow fraction at longer times (inset shows the splitting of the slow peak into two peaks at t=220 sec).

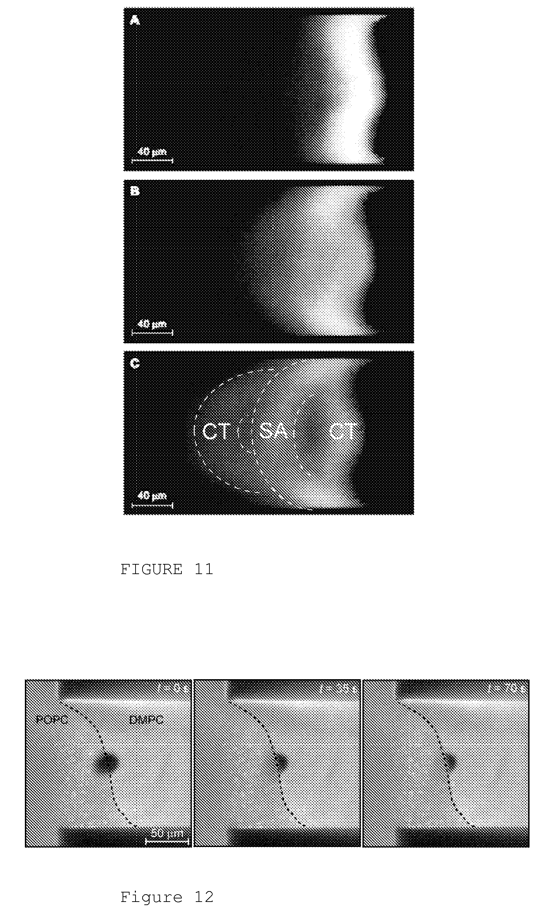

A second example demonstrates the separation of two different proteins. Figure 11 shows the overlaid fluorescence micrographs of streptavidin labelled with Cy3, (streptavidin-Cy3) and CTB-FITC molecules, both associated to a substantially planar surface supported lipid bilayer containing small fractions of biotin- and GM1-lipids, being driven away from the bilayer edge with a bulk flow of 200 µL/min in the negative x-direction (from right to left in Figure 11). The micrographs are taken at 30 s time intervals. The high intensity region in the fluorescence micrograph in Figure 11A corresponds to a mixture of streptavidin-Cy3 and CTB-FITC in the surface supported lipid bilayer. The surface supported lipid bilayer contained 0.00125 wt% biotin-PE and 0.05 wt% GM1 to anchor the proteins. The micrographs show that streptavidin-Cy3 and CTB-FITC have different drift velocities in the surface supported lipid bilayer under the action of a hydrodynamic force, which results in efficient separation of the different membrane associated molecules.

### Fusion of two substantially planar surface supported lipid bilayers

Figure 12 shows a substantially planar surface supported lipid bilayer made from POPC that was driven from left to right by the hydrodynamic force against a substantially planar surface supported lipid bilayer made from 1,2-dimyristoyl-sn-glycero-3-phosphocholine (DMPC)to the right in the figure. The temperature was around 20 °C. The black spot in the center of the figure is an area of the fluorescently labeled lipids in the surface supported lipid bilayer that has been photobleached at t = 0. Note, that whereas the lipids in the fluid surface supported lipid bilayer made from POPC are mobile, resulting in recovery of the photobleached area in this region, this is not the case for the lipids in the surface supported lipid bilayer made from DMPC. Note also that the boundary between the two types of surface supported lipid bilayers is driven in the direction of the bulk flow (the dashed line in the figures correspond to the position of the boundary at t = 0).

### Examples

### Fabrication of the microfluidic device

The microfluidic channel was made of polydimethylsiloxane (PDMS) using the technique of replica molding. The channel consisted of four channel arms in the shape of a 1×1 cm cross. A master made of SU-8 (SU-8 2035, MicroChem Corp., Newton, MA), defining the channel, was made using conventional optical lithography. The dimensions of the master were determined using an optical surface profiler (Wyko NT 1100, Veeco Instruments Inc., Tucson, AZ) yielding a height of 113 µm and a width of 150 **µ**m for each channel. There was also a 3×3 mm connection area at the end of each channel arm (labeled 1-4 in Figure 2 and 3, to which the inlet and outlet tubing was connected. The channel also had periodically spaced pillars (50×50 **µ**m wide; spaced 50 µm apart) to prevent the roof from collapsing.

The PDMS replica was made from a mixture of 10:1 Sylgard 184 and curing agent (Dow Corning, Midland, MI), which was allowed to cure for at least one hour at 95°C. A glass slide (0.13-0.16 mm in thickness, Menzel-Gläser, Braunschweig, Germany) was cleaned in a (5:1:1 by volume) mixture of deionized Milli Q™ water (Millipore, Billerica, MA), 25% ammonia, and 30% hydrogen peroxide at 70°C for 15 minutes, after which the sample was thoroughly rinsed with Milli Q™ water and dried at 150°C for 30 min. To obtain a strong seal between the PDMS and the substrate, the two surfaces were cleaned with an oxygen plasma cleaner (PDC-32G Plasma Cleaner, Harrick Plasma, Ithaca, NY) before bonding the PDMS to the substrate by placing the two surfaces in contact. Heating at 95°C for 10 min improved the strength of the seal. The channels were filled with Milli Q™ water (through capillary forces) immediately after bonding, to ensure that the surfaces remained hydrophilic. Prior to bonding the glass and the PDMS, access holes had been made in the PDMS, with a 1.2 mm hollow steel tube, in the four connection areas. Finally, silicone tubes (inner diameter 1.5 mm) were glued to the access holes using a silicone adhesive (Elastosil A07, RTV-1 silicone rubber, Wacker Silicones, Munich, Germany), constituting the inlets and outlets of the device.

The microfluidic channel was cleaned after each experiment by flowing a 1-2 wt% sodium dodecyl sulfate solution (Sigma-Aldrich) through each channel arm, after which the sample was cleaned in an ultrasound bath for ~15 min. The channel was then thoroughly rinsed with Milli Q™ water and again placed in the ultrasound bath before reuse.

### Preparation of liposomes

Liposomes were prepared by extrusion through a 30 nm membrane (Whatman, Maidstone, UK) using an Avanti Mini-Extruder (Avanti Polar Lipids). Liposomes consisted of 1 palmitoyl-2-oleoyl-sn-glycero-3-phosphocholine (POPC) or (**1**) 1,2-dioleoyl-sn-glycero-3-phosphocholine (DOPC) and cholesterol at 60:40 mole% ratio, (**2**) DOPC and cholesterol at a 50:50 mole% ratio and (**3**) DOPC, 1,2-dioleoyl-sn-glycero-3-phosphoethanolamine (DOPE) and cholesterol at a 39:21:40 mole% ratio. All lipids were from Avanti Polar Lipids (Alabaster, AL).

Liposomes made of POPC were in some cases dye labelled with 1 wt% 2(12-(7-nitrobenz-2-oxa-1,3-diazol-4-yl)amino)dodecanoyl-1-hexadecanoyl-sn-glycero-3-phosphocholine (NBD C12-HPC).

Liposomes made of compositions (**1**), (**2**) and (**3**) were dye labelled 1 wt% lissamine-rhodamine B 1,2-dihexadecanoyl-sn-glycero-3-phosphoethanolamine (rhodamine-DHPE).

In some experiments, a small fraction (0.1 wt%) monosialoganglioside GM1 from bovine brain (GM1; Sigma-Aldrich, Stockholm, Sweden) was added to liposomes made of POPC.

In other experiments, a small fraction (0.025 wt%) of 1,2-dioleoyl-sn-glycero-3-phosphoethanolamine-N-(cap biotinyl) (biotin-PE; Avanti Polar Lipids) was added to liposomes made of POPC.

The buffer solution used to prepare the liposomes was a mixture of 100 mM NaCI (Sigma-Aldrich), 10 mM tris[hydroxymethyl]aminomethane (TRIS; VWR International, Stockholm, Sweden) and 1 mM ethylenediaminetetraacetic acid disodium salt dihydrate (EDTA; Sigma-Aldrich), with a pH of 8.0. The liposomes were diluted with the buffer solution to a total lipid concentration of 100 µg/mL before each experiment.

For the experiment with both biotin-PE and GM1 in the substantially planar surface supported lipid bilayer the liposome solution consisted of 50 µg/mL liposomes containing 0.0025 wt% biotin-PE and 50 µg/mL of liposomes containing 0.1 wt% GM1. Under the assumption that both types of liposomes form an substantially planar surface supported lipid bilayer equally fast this will result in an substantially planar surface supported lipid bilayer with 0.00125 wt% biotin-PE and 0.05 wt% GM1.

Cell membrane derived liposomes were made by incubating 3T3 fibroblast cells with rhodamine-DHPE labeled (1 wt%) POPC liposomes, leading to lipid uptake by the cells and consequently labelling of their lipid membranes . Liposomes were then made from cell-membrane fragments by extruding the labeled cells through 200 nm polycarbonate membranes. Water soluble proteins were removed using ultracentrifugation and filtering using centrifugal filters (Amicon® Ultra - 0.5 mL 100K, Millipore™, Carrigtwohill, Co. Cork, Ireland). The size distribution of the cell membrane derived liposome was measured using a nanoparticle tracking analyzer (NanoSight, UK). The average diameter was determined to 150±50 nm.

### Forming surface supported lipid bilayers

To access the microfluidic device, PTFE tubing (1/16" outer diameter, 0.17 or 0.5 mm inner diameter, VWR International) was inserted into the silicone inlet and outlet tubes of the channel. The frictional force was sufficient to hold the tubing in place. A 6-way selection valve (Upchurch Scientific, Oak Harbor, WA) was connected to the tube on connection at channel arm 1, and a second 6-way selection valve to the tube on connection at channel arm 4, in order to be able to inject and switch between different solutions. A surface supported lipid bilayer was formed in the left part of the channel by injecting a liposome solution of composition (1) into channel arm 1 and a buffer solution (10 mM NaCl, 1 mM TRIS and 0.1 mM EDTA, pH = 7.4 or 8.0) into channel arm 4, while the valves attached to channel arms 2 and 3 were open. The liposome solution will thus flow between the inlet of channel arm 1 and the outlets on channel arms 2 and 3, selectively forming an surface supported lipid bilayer in the left-hand part of the device only.

### Adsorption of surface supported liposomes in front of a surface supported lipid bilayer

After formation of the surface supported lipid bilayer and removal of excess liposomes by rinsing with buffer, the valves connected to arms 2 and 3 were closed. The surface supported lipid bilayer could now be moved from left to right by applying a bulk flow of buffer from inlet 1 to outlet 4. The bulk flow rate was controlled by a syringe pump (NE-1000, New Era Pump Systems Inc., Wantagh, NY) and was set to 250 µl/min. The liposomes (DOPC/cholesterol, DOPC/DOPE/cholesterol or cell membrane derived) to be fused with the formed surface supported lipid bilayer made from POPC was added through inlet 1 (via the 6-way selection valve) and adsorbed to the glass slide not covered with surface supported lipid bilayer.

### Driving a surface supported lipid bilayer against surface supported liposomes

By switching back to a buffer flow from inlet to outlet 4, the surface supported lipid bilayer could be driven (from inlet 1 to outlet 4) towards and fusing with the adsorbed liposomes in front of the surface supported lipid bilayer. (Figure 2)

### Accumulation and separation of membrane bound molecules

After formation of the substantially planar surface supported lipid bilayer from POPC liposomes, and removal of excess liposomes from the bulk solution by rinsing, protein solution was injected into channel arm 1. The protein streptavidin (SA), labeled with the fluorescent dye Cy3, were acquired from Sigma-Aldrich and bind to biotin-PE in the substantially planar surface supported lipid bilayer. The B ubunit of the protein cholera toxin, labeled with the fluorescent group fluorescein isothiocyanate (FITC), also acquired from Sigma-Aldrich, binds to GM1. The B subunits will form a pentamer in solution (here referred to as CTB), and thus each CTB complex can bind up to five GM1 molecules. For the surface supported lipid bilayer with GM1, 2 **µ**g/mL (~30 nM) CTB-FITC was injected. For the experiments with both biotin-PE and GM1 in the substantially planar surface supported lipid bilayer, 10 µg/mL SA-Cy3 and 2 µg/mL CT-FITC was injected. Binding of the fluorescently labeled protein molecules was monitored in real time using total internal reflection fluorescence (TIRF) microscopy.

The final intensity was observed to be 6.3±0.4 (n = 4) times higher when having 0.1 wt% GM1 in the surface supported lipid bilayer than when having 0.01 wt% GM1 in the surface supported lipid bilayer. The values are here given as the average value ± one standard deviation obtained from n measurements. For SA-Cy3 the quotient between the equilibrium intensity when having 0.025 wt% biotin-PE and 0.0025 wt% biotin-PE in the substantially planar surface supported lipid bilayer was 10.5±0.5 (n = 3). Thus, the amount of SA-Cy3 bound to the surface seems to scale linearly with the amount of biotin-PE in the substantially planar surface supported lipid bilayer, whereas for CTB-FITC either not all GM1 receptors on the surface binds to a CTB-FITC molecule or the amount of GM1 anchors per CTB-FITC molecule increases when increasing the surface coverage of GM1.

After the surface coverage of proteins reached equilibrium (~30 min) the channel was rinsed and the valves connected to arms 2 and 3 were closed. The substantially planar surface supported lipid bilayer with the proteins could now be moved from left to right in Figure 9 and 11 by applying a bulk flow of buffer solution (10 mM NaCl, 1 mM TRIS and 0.1 mM EDTA, pH = 8.0) from the inlet at arm 1 to the outlet at arm 4. The bulk flow rate was controlled by a syringe pump (NE-1000, New Era Pump Systems Inc., Wantagh, NY).

After accumulating the proteins at the bilayer front, the flow was reversed by turning the knobs of the two 6-way selection valves so that the tubing to connection area 1 was open and the tubing to connection area 4 was connected to a syringe injecting buffer solution at a fixed bulk flow rate, thus driving the accumulated proteins away from the bilayer edge. After the proteins had been driven back in the substantially planar surface supported lipid bilayer they could again be accumulated at the edge of the bilayer by reversing the bulk flow.

### The microscopy setup

The fluorescently labeled molecules were studied with an inverted Nikon Eclipse Ti-E microscope (Nikon Corporation, Tokyo, Japan), using an Andor iXon+ EMCCD camera (Andor Technology, Belfast, Northern Ireland), and a 60× magnification (NA = 1.49) oil immersion objective (Nikon Corporation). The acquired images consisted of 512x512 pixels with a pixel size of 0.38 × 0.38 **µ**m. To monitor the fluorescent molecules, a mercury lamp connected to the microscope using an optical fiber (Intensilight C-HGFIE; Nikon Corporation) was used together with a TRITC or a FITC filter cube (Nikon Corporation), depending on the dye studied.

### Fluorescence recovery after photobleaching (FRAP).

FRAP was used to determine the diffusivity of the labeled molecules in the lipid bilayer. All diffusivity measurements were performed with the bulk flow turned off. To create circularly symmetric bleached areas diode-pumped solid state lasers at either 475 nm (BWB-475-20E; B&W Tek Inc., Newark, DE) or at 532 nm (BWN-532-100E; B&W Tek Inc.) were used, depending on what dye the lipid bilayer contained. The acquired images were analyzed using the Hankel transform method, previously developed by us [Jönsson, P. et al, Biophys. J. 2008, (95) p5334]. A single exponential with an offset was fitted to the data yielding the diffusivity and immobile fraction.

## Claims

1. A method for fusing lipid bilayers comprising the steps of:
providing a supportive substrate having a surface;
providing a first lipid bilayer on said surface of said supportive substrate;
providing a second lipid bilayer on said surface of said supportive substrate; and
providing a force on at least said first lipid bilayer such that said first lipid bilayer is driven along said surface of said substrate towards said second lipid bilayer and said first lipid bilayer is fused with said second lipid bilayer.

2. A method according to claim 1, wherein said force is provided by providing a flow of a liquid along said first lipid bilayer.

3. A method according to claim 1, wherein said force is provided by a hydrodynamic flow or an electroosmotic flow, or by any combinations thereof.

4. A method according to any one of the preceding claims, wherein said first lipid bilayer is different from said second lipid bilayer with respect to at least one of structural conformation and molecular composition.

5. A method according to any one of the preceding claims, wherein said structural conformation of said first lipid bilayer is substantially planar.

6. A method according to any one of the preceding claims, wherein said second lipid bilayer is a vesicle.

7. A method according to any one of the preceding claims, wherein said surface of said substrate comprises a flow path.

8. A method according to any one of the preceding claims, wherein said substrate is made from one or more materials.

9. A method according to any one of the preceding claims, wherein said surface of said substrate is at least partially chemically or mechanically modified, in order to adapt interactions between the lipid bilayer and said surface of said substrate.

10. A method according to claim 9, wherein said surface of said substrate comprises an embedded sensor element.

11. A method according to any one of the preceding claims, wherein said surface of said substrate comprises a spacer molecule.

12. A method according to claim 11, wherein said spacer molecule is positioned between said surface of said substrate and to at least one of said first lipid bilayer and said second lipid bilayer.

13. A method according to any one of the preceding claims, wherein said surface of said substrate comprises a barrier.
